# EUROPEAN PATENT APPLICATION

(11) **EP 4 091 637 A1**
(43) Date of publication of application: **23.11.2022**
(21) Application number: 21741013.3
(22) Date of filing: 15.01.2021
(51) Int. Cl.: A61L 2/20, A61L 9/015, A61L 101/02, A61L 101/06, A61L 101/32, A61L 101/50, A61L 101/36

(54) **KIT FOR CHLORINE DIOXIDE FUMIGATION**

(30) Priority: 17.01.2020 KR 20200006719; 09.12.2020 KR 20200171416
(71) Applicant: Jin, Nam-seop, Seoul 06326 (KR)
(72) Inventor: Jin, Nam-seop, Seoul 06326 (KR)
(74) Representative: Herrero & Asociados, S.L.
(86) International application number: PCT/KR2021/000619
(87) International publication number: WO 2021/145732

(57) **Abstract**

The present invention relates to a kit for chlorine dioxide fumigation, a method for manufacturing same, and a method for using same. When the kit of the present invention is used, a high concentration of chlorine dioxide can be generated within a short time period without combustion and explosion. In addition, the kit is conveniently produced and packed as compared to existing disinfection kits, is easy to use, and produces a large amount of chlorine dioxide gas which is safer and has high purity, and thus can be effectively used in space disinfection.

## Description

### Technical Field

This application claims priority to Korean Patent Application No. 10-2020-0006719 filed in the Korean Intellectual Property Office on 17 January 2020 and Korean Patent Application No. 10-2020-0171416 filed in the Korean Intellectual Property Office on 9 December 2020, the disclosures of which are incorporated herein by reference.

The present disclosure relates to a kit for chlorine dioxide fumigation, a manufacturing method therefor, and a method for use thereof.

### Background Art

A fumigator (fumigating device) is used for the disinfection or the like of the interior of a building. The fumigator has advantages of showing a more effective effect in a closed room due to fumigating active ingredients volatilized into the air, being able to disinfect areas not reached by human hands by allowing fumigated chemicals to act on every corner of the room, and causing no damage to indoor furniture or equipment by the moisture of a disinfection solution, unlike spray disinfection.

As such, the fumigator achieves a disinfecting effect by volatilizing fumigating ingredients into the air, and thus the fumigating ingredients applicable to the fumigator need to be volatilized into the air by heat and to exhibit disinfection activity even when volatilized in a gas state.

Chlorine dioxide (ClO₂) is a substance that performs disinfection, deodorization, and bleaching actions in the form of an aqueous solution or gas, and can be used for disinfection and deodorization for medical facilities, such as spaces where infectious diseases break out, patient's hospitalization spaces, microbial laboratory spaces, large-sized medical equipment, and laboratory animal breeding spaces, agriculture and stockbreeding facilities, such as barns, greenhouses, food storage warehouses, and animal and plant quarantine facilities, insides and outsides of various types of transportation means, and the like.

Specifically, products for disinfecting surroundings of life and apparatuses have been developed and marketed by using a method wherein sodium chlorite (NaClO₂) is processed into a gel form or the like and then a low concentration of chlorine dioxide gas is generated through a physicochemical reaction. However, existing products including these gel type products cannot generate a large amount of chlorine dioxide at high concentrations within a short period of time. The reason is that sodium chlorite is a very reactive substance and thus causes combustion, flaming, or explosion of reactants during the generation of chlorine dioxide.

Therefore, there is a need for the development of a fumigation (smoke disinfection) method that can temporarily generate a high concentration of chlorine dioxide more safely and conveniently by controlling such a radical reaction, and an improved fumigation kit that is simple to manufacture and use also needs to be developed.

Throughout the entire specification, many patent documents are referenced and their citations are represented. The disclosures of cited papers and patent documents are entirely incorporated by reference into the present specification, and the level of the technical field within which the present disclosure falls and details of the present disclosure are explained more clearly.

### Disclosure of Invention

### Technical Problem

The present inventors have attempted to develop a method for generating a high concentration of chlorine dioxide within a short period of time without combustion or explosion. As a result, the present inventors established that a high concentration of chlorine dioxide can be generated within a short period of time without combustion or explosion when a predetermined amount of sodium chlorite is mixed with a predetermined concentration of a sugar solution diluted in water and then reaction heat is applied thereto using a separate thermogenic agent, and also confirmed that a kit for chlorine dioxide fumigation can be manufactured through a simple configuration from manufacturing and packaging of a product by the addition of small amounts of an acid and sugars even without a thermogenic agent, and thus completed the present disclosure.

An aspect of the present disclosure is to provide a kit for chlorine dioxide fumigation.

Another aspect of the present disclosure is to provide a method for using a kit for chlorine dioxide fumigation.

Still another aspect of the present disclosure is to provide a method for manufacturing a kit for chlorine dioxide fumigation.

Other purposes and advantages of the present disclosure will become more obvious when taken with the following detailed description of the disclosure, claims, and drawings.

### Solution to Problem

In accordance with an aspect of the present disclosure, there is provided a kit for chlorine dioxide fumigation, the kit including a sugar, water, and sodium chlorite.

As used herein, the term "fumigation" refers to a process in which active ingredients are volatilized in a gas state into the air to disinfect (sterilize, bleach, deodorize, and the like) a certain space or area.

In an embodiment of the present disclosure, the sugar is a sugar powder or a liquid sugar.

In an embodiment of the present disclosure, the sugar and water are mixed to form a sugar diluted solution.

In an embodiment of the present disclosure, the sugar diluted solution is obtained by diluting a sugar powder or a liquid sugar in water at a weight ratio of 1:0.1-10.

In an embodiment of the present disclosure, the sugar may be glucose, sugar, lactose, maltose, fructose, an oligosaccharide (e.g., fructooligosaccharide, isomaltooligosaccharide, galactooligosaccharide, maltooligosaccharide, xylooligosaccharide, gentiooligosaccharide, etc.), dextrin, and/or honey, but is not limited thereto.

In an embodiment of the present disclosure, the sodium chlorite and the sugar diluted solution are contained at a weight ratio of 1:0.1 to 1:10.

In an embodiment of the present disclosure, the kit further includes a heating inducer (or a heating agent or a thermogenic agent).

In an embodiment of the present disclosure, the heating inducer may include aluminum, calcium oxide, calcium carbonate, sodium carbonate, sodium hydroxide, a metal halide (e.g., sodium chloride, potassium chloride, magnesium chloride, calcium chloride, ferrous chloride, ferric chloride, cupric chloride, manganese chloride, zinc chloride, cuprous chloride, etc.), a metal sulfate (e.g., potassium sulfate, sodium sulfate, magnesium sulfate, calcium sulfate, copper sulfate, ferrous sulfate, ferric sulfate, manganese sulfate, etc.), an acetate (e.g., sodium nitrate, potassium nitrate, a nitrate, sodium acetate, etc.) and/or a carbonate (e.g., ferrous carbonate, etc.), and may include an iron powder, activated carbon, water, and the like, but is not limited thereto.

The heating inducer of the present disclosure may generate heat by a reaction, such as (4Fe+3O₂ -> 2Fe₂O₃ + heat) when using an oxidation reaction of iron, or may generate heat by a reaction such as (CaO + 2H₂O -> Ca(OH)₂ + 2H₂ + 1.5 kcal) when using a reaction of calcium oxide, but is not limited thereto.

In an embodiment of the present disclosure, the kit may be manufactured by including:
an A1 unit containing a sugar and water;
an A2 unit containing sodium chlorite; and
a B unit containing a heating inducer,
the A1 unit, the A2 unit, and the B unit being separately packaged.

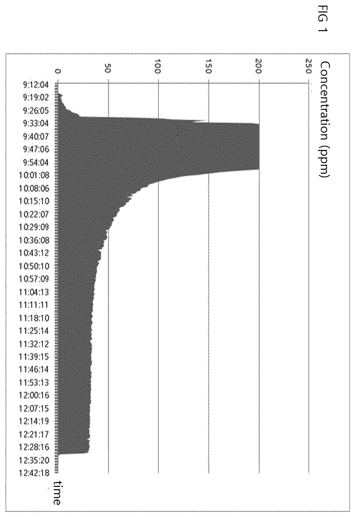

According to one example of the present disclosure, a sugar diluted solution was prepared and mixed with sodium chlorite, and then a heating inducer and water were added thereto to generate heat. As a result, the heated mixture reacted without combustion or explosion to generate chlorine dioxide gas (see FIG. 1).

That is, the kit for chlorine dioxide fumigation of the present disclosure can generate chlorine dioxide gas by a method in which a sugar diluted solution is mixed with sodium chlorite and then the mixture is allowed to react by using a heating inducer to apply heat thereto.

Therefore, the A1 unit, the A2 unit, and the B unit of the kit for chlorine dioxide fumigation of the present disclosure may be included while separately packaged, and the A2 unit may be added to the A1 unit before the B unit.

According to the present disclosure, the Al unit may contain the sugar diluted solution.

The sugar diluted solution may be prepared by dissolving a sugar powder or a liquid sugar in water at a weight ratio of 1:0.1-10, 1:0.1-9, 1:0.1-8, 1:0.1-7, 1:0.1-6, 1:0.1-5, 1:0.1-4, 1:0.1-3, 1:1-10, 1:2-10, 1:3-10, 1:3-9, 1:3-8, 1:3-7, 1:3-6, 1:3-5, 1:3, or 1:1, followed by dissolution.

Specifically, the sugar diluted solution may be prepared by adding a sugar powder and water at a weight ratio of 1:0.1-10 or dissolving a liquid sugar in water at a weight ratio of 1:0.1-5.

As for the sugar diluted solution, when the weight ratio between the sugar powder (or the liquid sugar) and water exceeds the above ranges, the reactants (the sugar diluted solution and sodium chlorite) may react violently to be boiled over or be combusted, and the viscosity of the sugar diluted solution is increased to fail the homogeneous mixing of the sugar diluted solution and sodium chlorite and to cause a partial excessive reaction of sodium chlorite, and thus the reaction liquid may spurt out of the container. When the weight ratio between the sugar powder (or the liquid sugar) and water is less than the above ranges, the reaction may slow down and may not start.

According to the present disclosure, the A2 unit contains sodium chlorite, wherein sodium chlorite and the sugar diluted solution may be contained at a weight ratio of 1:0.1-10, 1:0.5-10, 1:1-10, 1:1-9, 1:1-8, 1:1-7, 1:1-6, 1:1-5, 1:2-10, 1:3-10, 1:3-9, 1:3-8, 1:3-7, 1:3-6, 1:3-5, or 1:4.

When the weight ratio between sodium chlorite and the sugar diluted solution exceeds the above ranges, the reaction may not start since the amount of water contained in the sugar diluted solution is excessive. When the weight ratio between sodium chlorite and the sugar diluted solution is less than the above ranges, the units may not be mixed since the amount of sodium chlorite is excessive.

According to the present disclosure, the B unit contains a heating inducer, wherein the heating inducer may include aluminum, calcium oxide, calcium carbonate, sodium carbonate, sodium hydroxide, a metal halide (e.g., sodium chloride, potassium chloride, magnesium chloride, calcium chloride, ferrous chloride, ferric chloride, cupric chloride, manganese chloride, zinc chloride, cuprous chloride, etc.), a metal sulfate (e.g., potassium sulfate, sodium sulfate, magnesium sulfate, calcium sulfate, copper sulfate, ferrous sulfate, ferric sulfate, manganese sulfate, etc.), an acetate (e.g., sodium nitrate, potassium nitrate, a nitrate, sodium acetate, etc.) and/or a carbonate (e.g., ferrous carbonate, etc.); an iron powder, activated carbon, water, and the like, but is not limited thereto.

The heating inducer may be used by being packaged in a material through which water passes, such as nonwoven fabric, cotton wool, or gauze. However, in order to prevent an exothermic reaction before using the kit, the outermost packaging of a product surrounding the material is attained such that the heating inducer can be stored in a vacuum and moisture-free state.

In a case where the heating inducer is added, in a powder state without being packaged, to the mixture of the sugar diluted solution and sodium chlorite, the powder may be scattered and thus the generation of heat may not occur immediately or the generation of heat may be weak. Therefore, the heat inducer needs to be packaged in a material through which water can pass, so that the generation of heat is concentrated on the mixture. In particular, the packaging of the heating inducer in compressed cotton wool resulted in an excellent exothermic reaction.

The weight of the heating inducer may be determined by testing according to the volume of the mixture of the A1 unit and the A2 unit and the size of the reaction container, and is not included in direct proportion to the weights of the A1 unit and the A2 unit.

The initial reaction heat required for the reaction of the A1 unit and the A2 unit is provided by the heat generated by the B unit, and when the A units start to produce chlorine dioxide gas by a chemical reaction in the reaction container, the reaction of the mixture can be automatically kept by the reaction heat generated during the reaction.

In the kit for chlorine dioxide fumigation of the present disclosure, the heating inducer as described above is used as an inducer for an initial exothermic reaction, so that the reaction rate of the sugar diluted solution and sodium chlorite is adjusted to prevent high heat, flaming, or explosion caused by the reaction of the two substances.

The kit for chlorine dioxide fumigation of the present disclosure may further include, in addition to the above-described ingredients, a foaming agent, an insecticidal ingredient, a pest repellent ingredient, a deodorizing ingredient, an incense, or a combination thereof.

The kit for chlorine dioxide fumigation of the present disclosure can be applied to various fumigators. The fumigators are devices that perform a disinfection action by volatilizing fumigation ingredients into the air. The fumigators may be fabricated by a method wherein the kit for fumigation of the present disclosure is provided in various fumigators known in the art.

In one embodiment of the present disclosure, the kit further includes a heating container.

In a specific embodiment of the present disclosure, the heating container is formed of a material that is not deformed by the reaction heat, such as heat-resistant plastic, metal, glass, or ceramic. The inside temperature of the heating container may be increased by applying heat to the heating container using a separate heating means that is not limited to the type thereof, such as an alcohol lamp or a gas burner, or the inside temperature of the heating container may be increased by providing a heating means, such as a heating wire, inside the heating container. The inside temperature of the heating container may be increased to about 80°C to about 150°C.

In one embodiment of the present disclosure, the kit further includes an acid.

As described in one embodiment herein, when sodium chlorite is added and mixed with the solution containing a sugar, an acid, and water in the present disclosure, chlorine dioxide gas is very actively generated to diffuse into the entire space where the reaction occurs.

In an embodiment of the present disclosure, the acid may be at least one organic acid selected from the group consisting of citric acid, lactic acid, acetic acid, formic acid, tartaric acid, oxalic acid, ascorbic acid, uric acid, butyric acid, stearic acid, and propionic acid.

In an embodiment of the present disclosure, the acid may be at least one inorganic acid selected from the group consisting of hydrochloric acid, nitric acid, phosphoric acid, sulfuric acid, boric acid, and hydrofluoric acid.

In an embodiment of the present disclosure, the weight ratio between water and the sugar may be 10:0.1-10.

In a specific embodiment of the present disclosure, the weight ratio between water and the sugar included in the kit for fumigation may be 10:0.1-100, 10:0.1-90, 10:0.1-80, 10:0.1-70, 10:0.1-60, 10:0.1-50, 10:0.1-40, 10:0.1-30, 10:0.1-20, 10:0.1-10, 10:0.1-5, or 10:0.1-1; 10:0.5-100, 10:0.5-90, 10:0.5-80, 10:0.5-70, 10:0.5-60, 10:0.5-50, 10:0.5-40, 10:0.5-30, 10:0.5-20, 10:0.5-10, 10:0.5-5, or 10:0.5-1; 10:1-100, 10:1-90, 10:1-80, 10:1-70, 10:1-60, 10:1-50, 10:1-40, 10:1-30, 10:1-20, or 10:1-10, and more specifically, 10:0.1-10, 10:0.1-9, 10:0.1-8, 10:0.1-7, 10:0.1-6, 10:0.1-5, 10:0.1-4, 10:0.1-3, 10:0.1-2, 10:0.1-1, or 10:1-10, 10:1-9, 10:1-8, 10:1-7, 10:1-6, 10:1-5, 10:1-4, 10:1-3, 10:1-2, 10:9, 10:8, 10:7, 10:6, 10:5, 10:4, 10:3, 10:2, 10:1, 10:0.5, or 10:0.1, but is not limited thereto.

In an embodiment of the present disclosure, the acid included in the kit for fumigation may be contained in water at 0.01-10 M.

In a specific embodiment of the present disclosure, the acid included in the kit for fumigation may be contained in water at 0.1-10, 0.5-10, 1-10, 2-10, 3-10, 4-10, 5-10, 6-10, 7-10, 8-10, or 9-10 M.

In a specific embodiment of the present disclosure, the acid included in the kit for fumigation may be contained in water at 0.1-10, 0.1-9, 0.1-8, 0.1-7, 0.1-6, 0.1-5, 0.1-4, 0.1-3, 0.1-2, 0.1-1, or 0.1-0.5 M.

In an embodiment of the present disclosure, the weight ratio between a mixture solution of the sugar, the acid, and water excluding sodium chlorite and sodium chlorite is 1:0.1 to 1:10.

In a specific embodiment of the present disclosure, the weight ratio between a mixture solution of the sugar, the acid, and water excluding sodium chlorite and sodium chlorite, which are included in the kit for fumigation, may be 1:0.1-10, 1:0.5-10, 1:1-10, 1:2-10, 1:3-10, 1:4-10, 1:5-10, 1:6-10, 1:7-10, 1:8-10, or 1:9-10.

In another specific embodiment of the present disclosure, the weight ratio between a mixture solution of the sugar, the acid, and water excluding sodium chlorite and sodium chlorite, which are included in the kit for fumigation, may be 1:0.1-10, 1:0.1-9, 1:0.1-8, 1:0.1-7, 1:0.1-6, 1:0.1-5, 1:0.1-4, 1:0.1-3, 1:0.1-2, 1:0.1-1, or 1:0.1-0.5.

In an embodiment of the present disclosure, the kit for fumigation includes 20 to 200 parts by weight of the sugar relative to 100 parts by weight of sodium chlorite. More specifically, the kit for fumigation may include, relative to 100 parts by weight of sodium chlorite, the sugar in 20-200 parts by weight, 30-200 parts by weight, 40-200 parts by weight, 50-200 parts by weight, 60-200 parts by weight; 20-180 parts by weight, 30-180 parts by weight, 40-180 parts by weight, 50-180 parts by weight, 60-180 parts by weight; 20-160 parts by weight, 30-160 parts by weight, 40-160 parts by weight, 50-160 parts by weight, 60-160 parts by weight; 20-140 parts by weight, 30-140 parts by weight, 40-140 parts by weight, 50-140 parts by weight, 60-140 parts by weight; 20-120 parts by weight, 30-120 parts by weight, 40-120 parts by weight, 50-120 parts by weight, 60-120 parts by weight; 20-100 parts by weight, 30-100 parts by weight, 40-100 parts by weight, 50-100 parts by weight, 60-100 parts by weight; 20-80 parts by weight, 30-80 parts by weight, 40-80 parts by weight, 50-80 parts by weight, 60-80 parts by weight; 20-70 parts by weight, 30-70 parts by weight, 40-70 parts by weight, 50-70 parts by weight, 60-70 parts by weight, or 20 parts by weight, 30 parts by weight, 40 parts by weight, 50 parts by weight, 60 parts by weight, or 70 parts by weight, but is not limited thereto.

In another embodiment of the present disclosure, sodium chlorite included in the kit for fumigation may be provided in a state of being dissolved in water. In such a case, the kit for fumigation of the present disclosure may separately include the sugar or the acid, which are ingredients to be involved in a chloride dioxide generation reaction upon contact with sodium chlorite.

In accordance with according to another aspect of the present disclosure, there is provided a method for using the kit for chlorine dioxide fumigation, the method including:
i) mixing the sugar diluted solution and sodium chlorite; and
ii) adding a heating inducer and water to the mixture to conduct an exothermic reaction and generate chloride dioxide gas.

Since the method for using a kit for chloride dioxide fumigation or the method for chlorine dioxide fumigation in accordance with other aspects of the present disclosure are common with the above-described kit for chloride dioxide fumigation in terms of the elements, such as a sugar diluted solution, sodium dioxide, a heating inducer, and water, and the contents thereof, the overlapping description therebetween is omitted to avoid the complexity of the present application.

In accordance with according to another aspect of the present disclosure, there is provided method for using the kit for chlorine dioxide fumigation, the method including:
i) mixing the sugar diluted solution and sodium chlorite; and
ii) placing the mixture in the heating container, followed by heating.

In an embodiment of the present disclosure, the heating container is formed of a material that is not deformed by the reaction heat, such as heat-resistant plastic, metal, glass, or ceramic. The inside temperature of the heating container may be increased by applying heat to the heating container using a separate heating means that is not limited to the type thereof, such as an alcohol lamp or a gas burner, or the inside temperature of the heating container may be increased by providing a heating means, such as a heating wire, inside the heating container.

The inside temperature of the heating container may be increased to about 80°C to about 150°C.

In an embodiment of the present disclosure, when the mixture of the sugar diluted solution and sodium chloride is placed and heated in the heating container, the mixture of the sugar and sodium chlorite reacts to generate chlorine dioxide like when the above-described heating agent is used. In particular, the heating agent, after a certain period of time, is consumed to result in the termination of the exothermic reaction, and thus chlorine dioxide cannot be further generated. However, the heating container allows the continuous generation of chlorine dioxide when the sugar diluted solution and sodium chlorite are additionally added in the heating container.

In accordance with still another aspect of the present disclosure, there is provided a method for using the kit for chlorine dioxide fumigation, the method including:
i) mixing the sugar, the acid, and water, followed by dissolution; and
ii) adding sodium chlorite to the mixture, followed by mixing, thereby generating chloride dioxide gas.

In the step of generating chlorine dioxide ii) of the present disclosure, chlorine dioxide gas is very actively generated and thus a high concentration of chloride dioxide gas is generated.

In the above step, a high heat of 100°C or higher is generated while a chlorine dioxide gas generation reaction occurs, and thus chlorine dioxide gas spreads quickly and easily around a space where the reaction occurs, thereby disinfecting a wide space quickly.

Furthermore, the kit for chlorine dioxide fumigation used in the present disclosure does not include a thermogenic agent, and thus the kit can generate chlorine dioxide gas with higher purity since a third component that may be generated due to a high heat of 100°C or higher is not generated from impurities contained in the thermogenic agent.

Since the method for using a kit for chloride dioxide fumigation in accordance with still another aspect of the present disclosure is common with the above-described kit for chloride dioxide fumigation in terms of the elements, such as a sugar, an acid, and water, and the contents thereof, the overlapping description therebetween is omitted to avoid the complexity of the present application.

In accordance with still another aspect of the present disclosure, there is provided a method for manufacturing a kit for chlorine dioxide fumigation, the method including:
i) mixing a sugar, an acid, and water, followed by dissolution, thereby preparing a first unit; and
ii) preparing a second unit containing sodium chlorite.

In an embodiment of the present disclosure, the first unit is characterized to contain an acid. In addition, the first unit does not contain a thermogenic agent and thus can store the other ingredients, except for sodium chlorite, while previously mixed, so that the kit of the present disclosure can be easily manufactured and stored compared with a conventional kit for chlorine dioxide fumigation developed by the present inventors.

In an embodiment of the present disclosure, the first unit and the second unit are separately packaged since chlorine dioxide gas is generated upon contact of the first unit and the second unit, failing to generate chlorine dioxide gas at any time when a user desires.

Since the method for manufacturing a kit for chloride dioxide fumigation in accordance with still another aspect of the present disclosure is common with the above-described kit for chloride dioxide fumigation in terms of the elements, such as a sugar, an acid, and water, and the contents thereof, the overlapping description therebetween is omitted to avoid the complexity of the present application.

### Advantageous Effects of Invention

The present disclosure relates to a kit for chlorine dioxide fumigation, a manufacturing method therefor, and a method for use thereof, and the kit of the present disclosure can generate a high concentration of chlorine dioxide without combustion or explosion within a short period of time, is simpler to produce and package compared with an existing disinfection kit, is easy to use, and can be advantageously used for space disinfection by producing a large amount of chlorine dioxide gas with high purity more safely.

### Brief Description of Drawings

FIG. 1 shows the results of measuring concentration of chloride dioxide gas generated by a fumigating method according to one example of the present disclosure. At concentrations exceeding 200 ppm, the maximum value was not confirmed due to exceeding the limit of detection.
FIG. 2 sequentially shows a method for using a kit according to one example of the present disclosure (1. add a sodium chlorite powder to a glucose solution; 2. add an thermogenic agent; 3. add purified water; 4. chlorine dioxide gas being generated).
FIG. 3 sequentially shows a method for using a kit according to another example of the present disclosure (1. add a sodium chlorite powder to a solution of glucose and citric acid; 2. chlorine dioxide gas being generated).

### Mode for Carrying out the Invention

Hereinafter, the present disclosure will be described in more detail with reference to exemplary embodiments. These exemplary embodiments are provided only for the purpose of illustrating the present disclosure in more detail, and therefore, according to the purpose of the present disclosure, it would be apparent to a person skilled in the art that these examples are not construed to limit the scope of the present disclosure.

### EXAMPLES

### Example 1: Manufacturing of inventive kit for chlorine dioxide fumigation 1

### 1) Preparation of sugar diluted solutions

Glucose, sugar, maltose, fructose, and an oligosaccharide in a powder form were dissolved in water such that the weight ratio between water and each sugar was 3:1, and starch syrup and honey in a liquid phase were mixed with water such that the weight ratio between water and each sugar was 1:1. In the following test, 150 ml of each of the prepared sugar diluted solutions was used.

### 2) Preparation of mixtures of sodium chlorite and sugar diluted solution

To beakers containing the sugar diluted solutions was added 100 g of a sodium chlorite powder, and then well mixed, and used immediately after mixing.

### 3) Addition of heating inducer

A cotton wool pack containing 13 g of a heating inducer was placed on each of the mixtures of sodium chlorite and the sugar diluted solution, and 60 ml of water was poured thereon, and then the mixtures were heated.

### 4) Confirmation of generating chlorine dioxide gas

The heated mixtures reacted to generate gas, and as a result of measurement using a chlorine dioxide measurement instrument (6000-CLO2, measurement limit: 200ppm), the diluted solution of every type of sugar provided in the test reacted with sodium chlorite to generate chlorine dioxide gas.

Specifically, chlorine dioxide gas was generated at 5 to 20 minutes after the addition of the thermogenic agent in cases of glucose, maltose, and starch syrup, and the gas was generated at 40 minutes after the addition of the thermogenic agent in cases of sugar, oligosaccharide, fructose, and honey. As a result of measuring chlorine dioxide gas, the gas was generated for each case up to the measurement limit, 200 ppm, of the measurement instrument.

On the basis of the results, the following test was conducted using a solution in which water and glucose were mixed at a weight ratio of 75:25.

### Test Example 1: Measurement of chlorine dioxide concentration

A measurement instrument (Wandi Gas tiger 6000-CLO2) was installed in a closed space of about 120m³ (4.2m x 5.7m x 5m). In a metal container (a circular metal can with a diameter of 13 cm and a height of 15 cm) disposed on the ground in a space 5 m away from the measuring instrument, 150 ml of a glucose diluted solution and 100 g of a sodium chlorite powder were added and mixed well, and then a cotton wool pack containing 13 g of a thermogenic agent was placed on the mixture, and 60 ml of water was poured over a thermogenic agent. Then, the measurement instrument was operated from the time when the thermogenic agent was added, to measure the concentration of chlorine dioxide gas every one minute.

As can be confirmed from FIG. 1, chlorine dioxide gas started to be measured 5 minutes after the initiation of the measurement, and reached a concentration of 200 ppm or more 22 minutes after the initiation of the measurement. Thereafter, the concentration was kept at 200 ppm or more for 24 minutes and then slowly decreased, and the concentration was kept at 50 ppm or more for up to 1 hour and 20 minutes and at 30 ppm even after 3 hours in the space of 120m³.

### Example 2: Manufacturing of inventive kit for chlorine dioxide fumigation 2

A kit for fumigation capable of chlorine dioxide fumigation even without a thermogenic agent for chlorine dioxide was manufactured as follows. In addition, the kit was manufactured to have the composition shown in Table 1 to investigate the degree of improvement compared with the kit for chlorine dioxide fumigation manufactured in Example 1.

**TABLE 1**

| Compositions of kit of Example 1 and kit of Example 2 | | | | | | |
|---|---|---|---|---|---|---|
| Compo sition | Sodium chlorite (g) | Glucose (g) | Thermoge nic agent (g) | Citric acid (g) | Purified water contained in sugar solution (g) | Total amount of purified water (g) |
| Exampl e 1 | 100 | 40 | 13 | - | 110 | 170 (110 g contained in sugar solution + 60 g added to thermogenic agent) |
| Exampl e 2 | 100 | 60 | - | 5 | 170 | 170 |

The packaging configurations of the kit of Example 1 and the kit of Example 2 are shown in Table 2.

**TABLE 2**

| Packaging configurations (manufacturing processes) of kit of Example 1 and kit of Example 2 | |
|---|---|
| Kit of Example1: | 1. Package sodium chlorite powder |
| | 2. Package sugar solution (glucose + purified water) |
| | 3. Package thermogenic agent |
| | 4. Package purified water for generation of heat |
| | 5. Package all contents |
| 2: Kit of Example 2: | 1. Package sodium chlorite powder |
| | 2.Package mixture solution of glucose and citric acid (glucose + citric acid + purified water) |
| | 3. Package all contents |

As shown in Tables 1 and 2, sodium chlorite, the glucose solution, the thermogenic agent, and purified water for generation of heat need to be separately packaged in the kit of Example 1. However, the inventive kit of Example 2 is simple to package since glucose, citric acid, and purified water can be mixed into one and thus merely sodium chlorite and the solution of glucose + citric acid + purified water need to be separately packaged.

### Test Example 2: Using of kits for chlorine dioxide fumigation of Examples 1 and 2

### 2-1. Method of using kit of Example 1

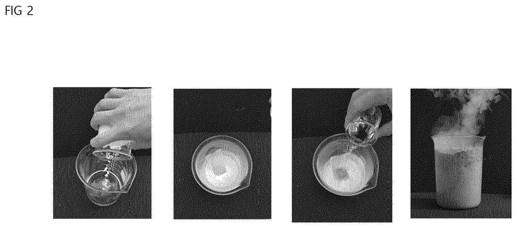

In the kit of Example 1, the separately packaged sodium chlorite powder was placed in a container containing the glucose solution, followed by homogeneous mixing, and then the thermogenic agent was added thereto. Thereafter, purified water was poured over the thermogenic agent, followed by an exothermic reaction. As a result, while the mixture was boiled, heat was generated and chlorine dioxide gas was generated. The method of using the kit of Example 1 is shown in Table 3 and FIG. 2.

### 2-2. Method of using kit of Example 2

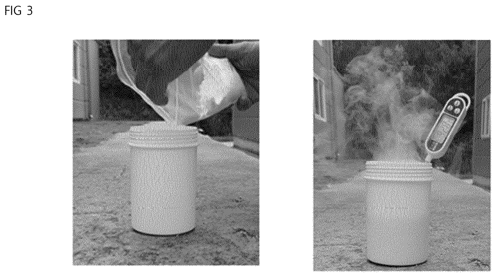

In the inventive kit of Example 2, the mixture solution containing glucose and citric acid was placed in a reaction container, and the sodium chlorite powder was added thereto. As a result, while the mixture was boiled, a high heat of 100°C or higher was generated, and chlorine dioxide gas was generated very quickly and simultaneously diffuses quickly throughout the space where the reaction occurred. The method of using the kit of Example 2 is shown in Table 3 and FIG. 3.

**TABLE 3**

| Methods of using kit of Example 1 and kit of Example 2 | |
|---|---|
| Kit of Example 1: | 1. Place 100 g of sodium chlorite powder in container containing 150 ml of glucose solution (40 g of glucose and 110 g of purified water) |
| | 2. Place thermogenic agent |
| | 3. Pour 60 g of purified water to thermogenic agent |
| Kit of Example 2: | 1. Place 100 g of sodium chlorite in container containing mixture solution of glucose and citric acid (60 g of glucose, 5 g of citric acid, and 170 g of purified water) |

As shown in Table 3 and FIGS. 2 and 3, the kit of Example 1 containing the thermogenic agent needed to pass through four steps: i) placing the glucose solution in the container, ii) adding sodium chlorite, iii) adding the thermogenic agent, and iv) adding purified water for an exothermic reaction.

However, the kit of Example 2 can generate chlorine dioxide gas through only two steps of: i) placing the mixture solution of glucose and citric acid in the container; and ii) adding sodium chlorite, and thus is simpler to use than the kit of Example 1.

As described above, the kit of Example 2 is simple for product production and packaging and is also simple to use, and furthermore, the kit of Example 2 is safer since there is no need to use a thermogenic agent and thus impurities that may be generated from the thermogenic agent is unlikely to be contained in the fumigated chlorine dioxide gas.

In the kit of Example 2, a high concentration of chloride dioxide gas is generated while chlorine dioxide gas is generated very actively, and a high heat of 100°C or higher is generated while the reaction occurred, and thus the chlorine dioxide gas could diffuse very quickly and easily throughout the space where the reaction occurred, thereby disinfecting a wide space quickly.

Accordingly, the kit for fumigation of Example 2 can be used very advantageously for space disinfection.

Although the present disclosure has been described in detail with reference to the specific features, it will be apparent to those skilled in the art that this description is only for a preferred embodiment and does not limit the scope of the present disclosure.

## Claims

1. A kit for chlorine dioxide fumigation, the kit comprising a sugar, water, and sodium chlorite.

2. The kit of claim 1, wherein the sugar is a sugar powder or a liquid sugar.

3. The kit of claim 1, wherein the sugar and water are mixed to form a sugar diluted solution.

4. The kit of claim 3, wherein the sugar diluted solution is prepared by diluting a sugar powder or a liquid sugar in water at a weight ratio of 1:0.1-10.

5. The kit of claim 1, wherein the sugar is at least one selected from the group consisting of glucose, sugar, lactose, maltose, fructose, an oligosaccharide, dextrin, and honey.

6. The kit of claim 3, wherein sodium chlorite and the sugar diluted solution are contained at a weight ratio of 1:0.1 to 1:10.

7. The kit of any one of claims 1 to 6, further comprising a heating inducer.

8. The kit of claim 7, wherein the kit comprises:
an A1 unit containing a sugar and water;
an A2 unit containing sodium chlorite; and
a B unit containing a heating inducer,
the A1 unit, the A2 unit, and the B unit being separately packaged.

9. The kit of any one of claims 1 to 6, further comprising a heating container.

10. The kit of any one of claims 1 to 6, further comprising an acid.

11. The kit of claim 10, wherein the acid is at least one organic acid selected from the group consisting of citric acid, lactic acid, acetic acid, formic acid, tartaric acid, oxalic acid, ascorbic acid, uric acid, butyric acid, stearic acid, and propionic acid.

12. The kit of claim 10, wherein the acid is at least one inorganic acid selected from the group consisting of hydrochloric acid, nitric acid, phosphoric acid, sulfuric acid, boric acid, and hydrofluoric acid.

13. The kit of claim 10, wherein the weight ratio between water and the sugar is 10:0.1-10.

14. The kit of claim 10, wherein the acid is contained in water at 0.01 to 10 M.

15. The kit of claim 10, wherein the weight ratio between a mixture solution of the sugar, the acid, and water excluding sodium chlorite and sodium chlorite is 1:0.1 to 1:10.

16. The kit of claim 10, wherein the kit comprises 20 to 200 parts by weight of the sugar relative to 100 parts by weight of sodium chlorite.

17. A method for using the kit for chlorine dioxide fumigation of claim 7 or 8, the method comprising:
i) mixing the sugar diluted solution and sodium chlorite; and
ii) adding a heating inducer and water to the mixture to conduct an exothermic reaction and generate chloride dioxide gas.

18. A method for using the kit for chlorine dioxide fumigation of claim 9, the method comprising:
i) mixing the sugar diluted solution and sodium chlorite; and
ii) placing the mixture in the heating container, followed by heating.

19. A method for using the kit for chlorine dioxide fumigation of any one of claims 10 to 16, the method comprising:
i) mixing the sugar, the acid, and water, followed by dissolution; and
ii) adding sodium chlorite to the mixture, followed by mixing, thereby generating chloride dioxide gas.
